(19) Europäisches Patentamt
European Patent Office
Office européen des brevets

(11) Veröffentlichungsnummer: **0 283 898**
**A2**

(12) # EUROPÄISCHE PATENTANMELDUNG

(21) Anmeldenummer: **88104043.0**

(51) Int. Cl.4: **C07C 143/42** , G03F 7/10

(22) Anmeldetag: **15.03.88**

(30) Priorität: **24.03.87 US 29843**

(43) Veröffentlichungstag der Anmeldung:
**28.09.88 Patentblatt 88/39**

(84) Benannte Vertragsstaaten:
**AT CH DE FR GB IT LI NL**

(71) Anmelder: **HOECHST CELANESE CORPORATION**
**Route 202-206 North**
**Somerville, N.J. 08876(US)**

(72) Erfinder: **Berner, Paul, Dr.**
**7 Aurora Road**
**East Greenwich Rhode Island 02818(US)**

(74) Vertreter: **Euler, Kurt Emil, Dr. et al**
**HOECHST AG - Werk KALLE Patentabteilung**
**Postfach 3540 Rheingaustrasse 190**
**D-6200 Wiesbaden(DE)**

(54) **Verfahren zur Herstellung von Aryldiazidsulfonsäuren.**

(57) Es wird ein Verfahren zur Herstellung von lichtempfindlichen Aryldiazidsulfonsäuren ausgehend von mit mindestens einer Hydroxylgruppe substituierten Arylsulfonsäure beschrieben, das dadurch gekennzeichnet ist, daß während der Reaktionsabfolge, die aus

a) Nitrosierung der mindestens eine Hydroxylgruppe enthaltende Arylsulfonsäure,

b) Reduktion der entstehenden Nitrosoverbindung im alkalischen pH-Bereich und Umwandlung in ein Sulfamat-Derivat,

c) Mischen des Sulfamat-Derivates mit einem Diazotierungsreagenz und

d) Ansäuern dieser Mischung zur Bildung der Aryldiazidsulfonsäure besteht, das nach jedem Reaktionsschritt entstehende und zur Weiterreaktion geeignete Reaktionsprodukt in Lösung verbleiben kann.

Der besondere Vorteil dieses Verfahrens besteht darin, daß die bei der Reaktion entstehenden Nebenprodukte durch Filtration bzw. Zentrifugation aus der Reaktionslösung abgetrennt werden können. Die Einstufigkeit des Verfahrens gewährleistet eine hohe Wirtschaftlichkeit des Verfahrens; die Gesamtausbeute der entstehenden Aryldiazidsulfonsäuren ist sehr hoch.

EP 0 283 898 A2

## Verfahren zur Herstellung von Aryldiazidsulfonsäuren

Die vorliegende Erfindung betrifft ein Verfahren zur Herstellung von lichtempfindlichen Aryldiazidsulfonsäuren, ausgehend von mit mindestens einer Hydroxylgruppe substituierten Arylsulfonsäure.

Aryldiazidsulfonsäuren werden als lichtempfindliche Verbindungen in Photoresistgemischen verwendet (J. Kosar, "Light Sensitive Systems", John Wiley & Sons, New York, Kapitel 7.4, 1965). Ihre Herstellung geht aus von einer mit Hydroxylgruppen substituierten Benzol-oder Naphthalinsulfonsäure, die mit Natriumnitrit in verdünnter wäßriger Säure nitrosiert wird. Die entstehende Nitrosoverbindung wird als Niederschlag isoliert, und nicht umgesetzte Ausgangsstoffe und Verunreinigungen werden durch Waschen entfernt. Anschließend wird die Nitrosoverbindung mit Natriumborhydrid oder Natriumdithionit zu einer Aminoverbindung reduziert. Die Aminoverbindung wird als Niederschlag abgetrennt; nicht umgesetzte Ausgangsstoffe und Verunreinigungen werden durch Waschen entfernt. Anschließend muß die Aminoverbindung erneut in Wasser suspendiert werden, um bei niedrigem pH-Wert in Kontakt mit Natriumnitrit die Diazidverbindung zu bilden.

Der Nachteil des oben beschriebenen Verfahrens ist dessen Mehrstufigkeit und die Notwendigkeit, die in wäßriger Lösung entstehenden und gehaltenen Zwischenprodukte vom jeweils als Niederschlag entstehenden Hauptprodukt zu isolieren und zu reinigen. Damit verbunden sind hohe Produktionskosten, niedrige Ausbeute und eine Produktreinheit, die zu wünschen übrig läßt.

Aufgabe der Erfindung war es daher, ein Verfahren zur Herstellung von lichtempfindlichen Aryldiazidsulfonsäuren bereitzustellen, das die bestehenden Nachteile des Verfahrens des Standes der Technik nicht aufweist.

Gelöst wird die Aufgabe durch ein Verfahren zur Herstellung von lichtempfindlichen Aryldiazidsulfonsäuren ausgehend von mit mindestens einer Hydroxylgruppe substituierten Arylsulfonsäure, dadurch gekennzeichnet, daß während der Reaktionsabfolge, die aus

a) Nitrosierung der mindestens eine Hydroxylgruppe enthaltenden Arylsulfonsäure,

b) Reduktion der entstehenden Nitrosoverbindung im alkalischem pH-Bereich und Umwandlung in ein Sulfamat-Derivat,

c) Mischen des Sulfamat-Derivates mit einem Diazotierungsreagenz und

d) Ansäuern dieser Mischung zur Bildung der Aryldiazidsulfonsäure besteht, das nach jedem Reaktionsschritt entstehende und zur Weiterreaktion geeignete Reaktionsprodukt in Lösung verbleiben kann.

Das erfindungsgemäße Verfahren bietet den überraschen den Vorteil, daß nicht das nach jedem Reaktionsschritt entstehende Hauptprodukt als Feststoff anfällt und deshalb abgetrennt werden muß, sondern daß die Nebenprodukte und Verunreinigungen als Feststoffe anfallen, und dadurch besser z.B. durch Filtration abgetrennt werden können. Dadurch wird auch die Einstufigkeit der Gesamtreaktion gewährleistet. Einher mit dieser Verfahrensabfolge gehen eine hohe Produktivität des erfindungsgemäßen Verfahrens, d.h. niedrigere Kosten und eine wirtschaftliche Ausnutzung der Reaktionsapparatur. Völlig überraschend war zudem, daß durch das erfindungsgemäße Verfahren auch eine höhere Gesamtausbeute der entstehenden Aryldiazidsulfonsäuren erzielt werden kann. Dies spricht für eine hohe Produktreinheit bei gleichzeitig geringerem Anfall von Nebenprodukten und Verunreinigungen.

Nach dem erfindungsgemäßen Verfahren hergestellte Aryldiazidsulfonsäuren können durch die folgende allgemeine Formel I dargestellt werden:

$$R_x\text{—}A\text{—}(SO_3M)_y \,, \qquad (I)$$

worin

A eine Arylgruppe mit einem Oxo-Anion (0=) und einem Diazonium-Kation ($N_2$=), z.B. des Chinondiazids oder des Naphthochinondiazids,

R einen einwertigen Substituenten, der ein Halogenatom, eine Nitrogruppe oder eine niedere Alkylgruppe mit 1 bis 6 C-Atomen sein kann,

M ein Wasserstoffatom, ein ein-, zwei-oder dreiwertiges Metall oder ein organisches Ammoniumion,

x eine ganze Zahl von 0 bis 3 und

y eine ganze Zahl von 1 bis 3

bedeuten.

Die Aryldiazidsulfonsäuren der oben angegebenen Formel werden nach dem erfindungsgemäßen Verfahren aus mit Hydroxylgruppen substituierten Arylsulfonsäuren der allgemeinen Formel II

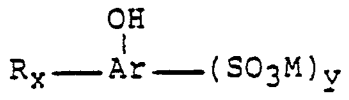

(II)

hergestellt, worin Ar einen Benzol-oder Naphthalinkern darstellt und R, M, x und y die oben angegebene Bedeutung haben.

Insbesondere haben die nach dem erfindungsgemäßen Verfahren herstellbaren Aryldiazidsulfonsäuren folgende allgemeine Formeln:

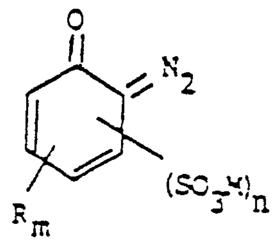

(III)

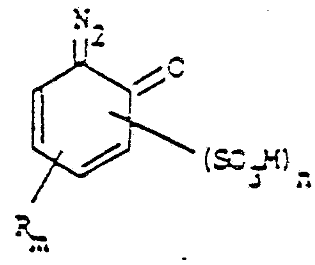

(IV)

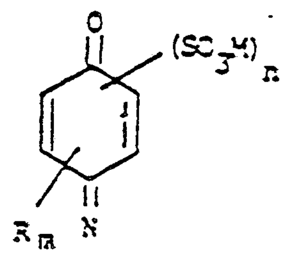

(V)

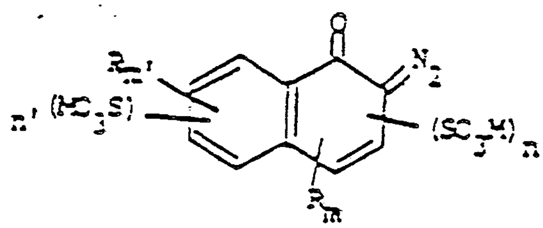

(VI)

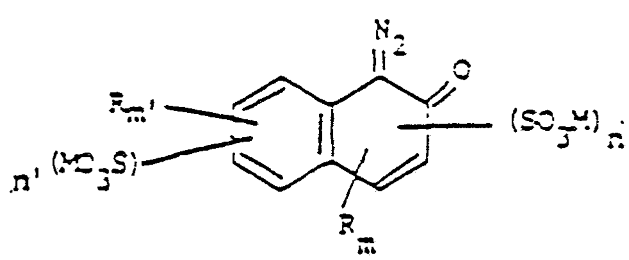

(VII)

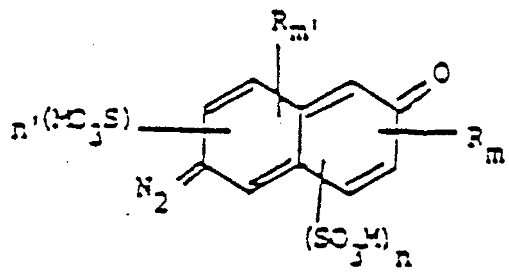

(VIII)

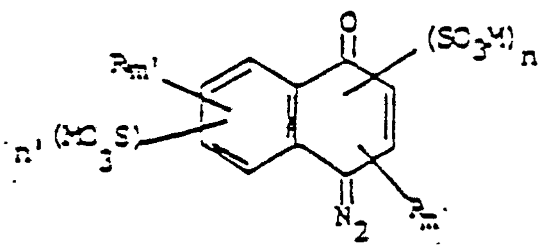

(IX)

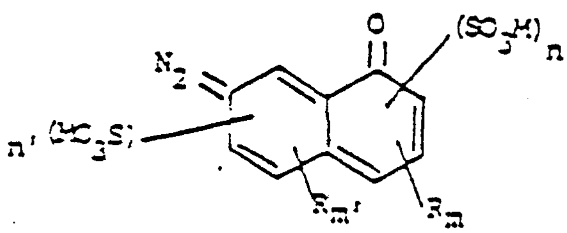

(X)

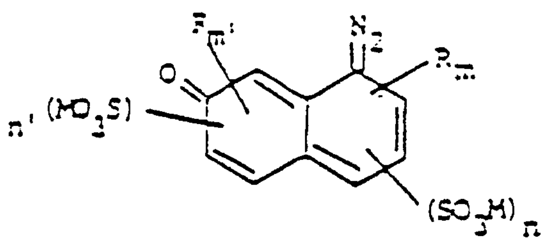

(XI)

In den allgemeinen Formeln III bis XI haben R und M die gleichen Bedeutungen, wie in den allgemeinen Formel I und II. m, m' und n, n' sind ganze Zahlen von 0 bis 3, die gleich oder verschieden sein können. Voraussetzung ist aber, daß mindestens eine ($SO_3M$)-Gruppe vorhanden ist.

Als besonders bevorzugte Beispiele werden genannt: Benzochinondiazidmonosulfonsäuren, Naphthochinondiazidmonosulfonsäuren, einschließlich der durch Nitro, Chlor, Brom oder Alkyl am Kern substituierten Derivate, sowie deren Natrium-, Kalium-, Magnesium-, Calcium-, Barium-, Aluminium-, Trimethylammonium-, Triethylammonium-, Pyridinium-oder N,N-Dimethylanilinsalze.

Im einzelnen werden genannt: 1,4-Benzochinon-4-diazid-2-sulfonsäure, 1,4-Benzochinon-4-diazid-3-sulfonsäure, 2-Methyl-1,4-benzochinon-4-diazid-5-sulfonsäure, 2-Methyl-1,4-benzochinon-4-diazid-5-sulfonsäure, 2-Isopropyl-1,4-benzochinon-4-diazid-3-sulfonsäure, 2-Chlor-1,4-benzochinon-4-diazid-5-sulfonsäure, 2-Chlor-1,4-benzochinon-4-diazid-6-sulfonsäure, 2-Brom-1,4-benzochinon-4-diazid-5-sulfonsäure, 2-Brom-1,4-benzochinon-4-diazid-6-sulfonsäure, 2-Nitro-1,4-benzochinon-4-diazid-5-sulfonsäure, 2,6-Dimethyl-1,4-benzochinon-4-diazid-3-sulfonsäure, 2,6-Dichlor-1,4-benzochinon-4-diazid-3-sulfonsäure, 2,6-Dibrom-1,4-benzochinon-4-diazid-3-sulfonsäure, 2-Chlor-6-nitro-1,4-benzochinon-4-diazid-3-sulfonsäure, 2-Fluor-1,4-benzochinon-4-diazid-5-sulfonsäure, 1,2-Benzochinon-2-diazid-3-sulfonsäure, 1,2-Benzochinon-2-diazid-4-sulfonsäure, 1,2-Benzochinon-2-diazid-5-sulfonsäure, 1,2-Benzochinon-2-diazid-6-sulfonsäure, 4-Nitro-1,2-benzochinon-2-diazid-5-sulfonsäure, 4-Chlor-1,2-benzochinon-2-diazid-5-sulfonsäure, 4-Brom-1,2-benzochinon-2-diazid-5-sulfonsäure, 6-Chlor-1,2-benzochinon-2-diazid-4-sulfonsäure, 6-Brom-1,2-benzochinon-2-diazid-4-sulfonsäure, 6-Chlor-1,2-benzochinon-2-diazid-5-sulfonsäure, 6-Brom-1,2-benzochinon-2-diazid-5-sulfonsäure, 6-Nitro-1,2-benzochinon-2-diazid-5-sulfonsäure, 4-Methyl-1,2-benzochinon-2-diazid-5-sulfonsäure, 4-Methyl-1,2-benzochinon-2-diazid-5-sulfonsäure, 5-Methyl-1,2-benzochinon-2-diazid-4-sulfonsäure, 3,5-Dichlor-1,2-benzochinon-2-diazid-4-sulfonsäure, 3,5,6-Trichlor-1,2-benzochinon-2-diazid-4-sulfonsäure, 4-Nitro-6-chlor-1,2-benzochinon-2-diazid-5-sulfonsäure, 1,2-Benzochinon-2-diazid-3,5-disulfonsäure,

1,2-Naphthochinon-2-diazid-3-sulfonsäure, 1,2-Naphthochinon-2-diazid-4-sulfonsäure, 1,2-Naphthochinon-2-diazid-5-sulfonsäure, 1,2-Naphthochinon-2-diazid-6-sulfonsäure, 1,2-Naphthochinon-2-diazid-7-sulfonsäure, 4-Chlor-1,2-naphthochinon-2-diazid-5-sulfonsäure, 3-Brom-1,2-naphthochinon-2-diazid-5-sulfonsäure, 4-Nitro-1,2-naphthochinon-2-diazid-3-sulfonsäure, 6-Nitro-1,2-naphthochinon-2-diazid-4-sulfonsäure, 6-Nitro-1,2-naphthochinon-2-diazid-5-sulfonsäure, 1,2-Naphthochinon-2-diazid-3,6-disulfonsäure, 1,2-Naphthochinon-2-diazid-4,6-disulfonsäure, 1,2-Naphthochinon-2-diazid-4,6,8-trisulfonsäure, 1,2-Naphthochinon-1-diazid-4-sul fonsäure, 1,2-Naphthochinon-1-diazid-5-sulfonsäure, 1,2-Naphthochinon-1-diazid-6-sulfonsäure, 1,2-Naphthochinon-1-diazid-7-sulfonsäure, 1,2-Naphthochinon-1-diazid-8-sulfonsäure, 3-Chlor-1,2-naphthochinon-1-diazid-5-sulfonsäure, 6-Chlor-1,2-naphthochinon-1-diazid-4-sulfonsäure, 8-Chlor-1,2-naphthochinon-1-diazid-4-sulfonsäure, 3-Brom-1,2-naphthochinon-1-diazid-4-sulfonsäure, 7-Brom-1,2-naphthochinon-1-diazid-4-sulfonsäure, 6,8-Dichlor-1,2-naphthochinon-1-diazid-4-sulfonsäure, 6-Nitro-1,2-naphthochinon-1-diazid-4-sulfonsäure, 5-Nitro-1,2-naphthochinon-1-diazid-6-sulfonsäure, 1,2-Naphthochinon-1-diazid-3,6-disulfonsäure, 1,2-Naphthochinon-1-diazid-4,6-disulfonsäure, 1,4-Naphthochinon-4-diazid-5-sulfonsäure, 1,4-Naphthochinon-4-diazid-7-sulfonsäure, 7-Chlor-1,4-naphthochinon-4-diazid-7-sulfonsäure, 1,4-Naphthochinon-4-diazid-5,7-disulfonsäure, 1,7-Naphthochinon-7-diazid-3-sulfonsäure, 1,7-Naphthochinon-7-diazid-3,6-disulfonsäure, 1,6-Naphthochinon-6-diazid-3-sulfonsäure, 2,6-Naphthochinon-6-diazid-1,4-disulfonsäure, 2-Nitro-1,4-naphthochinon-4-diazid-7-sulfonsäure und deren Natrium-, Kalium, Magnesium, Calcium-, Barium-, Aluminium,-Trimethylammonium-, Triethylammonium-, Pyridinium-oder N,N-Dimethylanilinsalze.

1,2-Benzochinon-2-diazid-4-sulfonsäure, 1,2-Benzochinon-2-diazid-5-sulfonsäure, 1,2-Naphthochinon-2-diazid-4-sulfonsäure, 1,2-Naphthochinon-2-diazid-5-sulfonsäure, 1,2-Naphthochinon-2-diazid-6-sulfonsäure, 1,2-Naphthochinon-2-diazid-7-sulfonsäure, 1,2-Naphthochinon-1-diazid-4-sulfonsäure, 1,2-Naphthochinon-1-diazid-5-sulfonsäure, 1,2-Naphthochinon-1-diazid-6-sulfonsäure und 1,2-Naphthochinon-1-diazid-7-sulfonsäure sowie deren Natrium-, Kalium, Calcium-oder Bariumsalze werden vorzugsweise eingesetzt.

Insbesondere bevorzugt sind 1,2-Naphthochinon-2-diazid-5-sulfonsäure, 1,2-Naphthochinon-2-diazid-4-sulfonsäure und 1,2-Naphthochinon-1-diazid-4-sulfonsäure sowie deren Natrium-, Kalium-, Calcium-oder Bariumsalze.

Die oben zusammengefaßten Aryldiazidsulfonsäuren werden nach dem erfindungsgemäßen Verfahren aus mit mindestens einer Hydroxylgruppe substituierten Benzolsulfonsäuren bzw. Naphthalinsulfonsäuren der allgemeinen Formel II hergestellt.

Beispiele dieser Säuren sind Hydroxybenzolmono-, -di-, und -trisulfonsäuren, Hydroxynaphthalinmono-, -di-und -trisulfonsäuren, und deren durch Nitro, Chlor, Brom oder Alkyl am Kern substituierten Verbindungen sowie die Natrium-, Kalium-, Magnesium-, Calcium-, Barium-, Aluminium-, Trimethylammonium-Triethylammonium-, Pyridinium-oder N,N-Dimethylammoniumsalze der Sulfonsäuren.

Im einzelnen werden als Beispiele dieser Säuren genannt:

1-Hydroxybenzol-2-sulfonsäure, 1-Hydroxybenzol-3-sul fonsäure, 2-Methyl-1-hydroxybenzol-5-sulfonsäure, 2-Methyl-1-hydroxybenzol-6-sulfonsäure, 2-Isopropyl-1-hydroxybenzol-3-sulfonsäure, 2-Chlor-1-hydroxybenzol-5-sulfonsäure, 2-Chlor-1-hydroxybenzol-6-sulfonsäure, 2-Brom-1-hydroxybenzol-5-sulfonsäure, 2-Brom-1-hydroxybenzol-6-sulfonsäure, 2-Nitro-1-hydroxybenzol-5-sulfonsäure, 2,6-Dimethyl-1-hydroxybenzol-3-sulfonsäure, 2,6-Dichlor-1-hydroxybenzol-3-sulfonsäure, 2,6-Dibrom-1-hydroxybenzol-3-sulfonsäure, 2-Chlor-6-nitro-1-hydroxybenzol-3-sulfonsäure, 2-Fluor-1-hydroxybenzol-5-sulfonsäure, 1-Hydroxybenzol-3-sulfonsäure, 1-Hydroxybenzol-4-sulfonsäure, 1-Hydroxybenzol-5-sulfonsäure, 1-Hydroxybenzol-6-sulfonsäure, 4-Nitro-1-hydroxybenzol-5-sulfonsäure, 4-Chlor-1-hydroxybenzol-5-sulfonsäure, 4-Brom-1-hydroxybenzol-5-sulfonsäure, 6-Chlor-1-hydroxybenzol-4-sulfonsäure, 6-Brom-1-hydroxybenzol-4-sulfonsäure, 6-Chlor-1-hydroxybenzol-5-sulfonsäure, 6-Brom-1-hydroxybenzol-5-sulfonsäure, 6-Nitro-1-hydroxybenzol-5-sulfonsäure, 4-Methyl-1-hydroxybenzol-5-sulfonsäure, 4-Methyl-1-hydroxybenzol-5-sulfonsäure, 5-Methyl-1-hydroxybenzol-4-sulfonsäure, 3,5-Dichlor-1-hydroxybenzol-4-sulfonsäure, 3,5,6-Trichlor-1-hydroxybenzol-4-sulfonsäure, 4-Nitro-6-chlor-1-hydroxybenzol-5-sulfonsäure,

1-Hydroxynaphthalin-3,5-disulfonsäure, 1-Hydroxynaphthalin-3-sulfonsäure, 1-Hydroxynaphthalin-4-sulfonsäure, 1-Hydroxynaphthalin-5-sulfonsäure, 1-Hydroxynaphthalin-6-sulfonsäure, 1-Hydroxynaphthalin-7-sulfonsäure, 4-Chlor-1-hydroxynaphthalin-5-sulfonsäure, 3-Brom-1-hydroxynaphthalin-5-sulfonsäure, 4-Nitro-1-hydroxynaphthalin-5-sulfonsäure, 6-Nitro-1-hydroxynaphthalin-4-sulfonsäure, 6-Nitro-1-hydroxynaphthalin-5-sulfonsäure, 1-Hydroxynaphthalin-3,6-disulfonsäure, 1-Hydroxynaphthalin-4,6-disulfonsäure, 1-Hydroxynaphthalin-4,6,8-trisulfonsäure, 1-Hydroxynaphthalin-4-sulfonsäure, 2-Hydroxynaphthalin-5-sulfonsäure, 2-Hydroxynaphthalin-6-sulfonsäure, 2-Hydroxynaphthalin-7-sulfonsäure, 2-Hydroxynaphthalin-8-sulfonsäure, 3-Chlor-1-hydroxynaphthalin-5-sulfonsäure, 6-Chlor-2-hydroxynaphthalin-4-sulfonsäure, 8-Chlor-2-hydroxynaphthalin-4-sulfonsäure, 3-Brom-2-hydroxynaphthalin-4-sulfonsäure, 7-Brom-2-hydroxynaphthalin-4-sulfonsäure, 6,8-Dichlor-2-hydroxynaphthalin-4-sulfonsäure, 6-Nitro-2-hydroxynaphthalin-4-sulfonsäure, 5-Nitro-2-hydroxynaphthalin-6-sulfonsäure, 2-Hydroxynaphthalin-3,6-disulfonsäure, 2-Hydroxynaphthalin-4,6-disulfonsäure, 1-Hydroxynaphthalin-5-sulfonsäure, 1-Hydroxynaphthalin-6-sulfonsäure, 1-Hydroxynaphthalin-7-sulfonsäure, 7-Chlor-1-hydroxynaphthalin-5,7-sulfonsäure,

1-Hydroxybenzol-4-sulfonsäure, 1-Hydroxybenzol-5-sulfonsäure, 1-Hydroxynaphthalin-4-sulfonsäure, 1-Hydroxynaphthalin-5-sulfonsäure, 1-Hydroxynaphthalin-6-sulfonsäure, 1-Hydroxynaphthalin-7-sulfonsäure, 2-Hydroxynaphthalin-4-sulfonsäure, 2-Hydroxynaphthalin-5-sulfonsäure, 2-Hydroxynaphthalin-6-sulfonsäure und 2-Hydroxynaphthalin-7-sulfonsäure

sowie deren Natrium-, Kalium-, Calcium-oder Bariumsal ze werden vorzugsweise eingesetzt. Insbesondere bevorzugt sind 1-Hydroxynaphthalin-5-sulfonsäure, 1-Hydroxynaphthalin-4-sulfonsäure und 1,2-Naphthochinon-1-diazid-4-sulfonsäure sowie deren Natrium-, Kalium-, Calcium-oder Bariumsalze.

In dem erfindungsgemäßen Verfahren wird eine mit mindestens einer Hydroxylgruppe substituierte Benzolsulfonsäure oder eine entsprechende Naphthalinsulfonsäure in einem Lösemittel, vorzugsweise in Wasser, gelöst. Die anschließende Nitrosierung (a) wird bevorzugt mit Natriumnitrit durchgeführt, das der angesäuerten wäßrigen Lösung der Sulfonsäure zugesetzt wird.

Die Nitrosierung kann bei einer Temperatur von 0 bis 40° C, vorzugsweise bei 5 bis 20° C, insbesondere bei 5 bis 15° C, durchgeführt werden. Während des Nitrosierungsschritts soll der pH-Wert des Reaktionsgemischs im Bereich von 1 bis 3 liegen und vorzugsweise 1 bis 2 betragen, insbesondere bei 1,5 liegen.

Die Nitrosierung kann erfolgen, indem eine wäßrige Lösung von Natriumnitrit in eine Lösung der mit Salzsäure angesäuerten mindestens eine Hydroxylgruppe enthaltenden aromatischen Sulfonsäure gegeben werden. Auf 1 Mol der aromatischen Sulfonsäure wird mindestens 1 Mol Natriumnitrit verwendet; im allgemeinen ist ein Überschuß des Nitrosierungsmittels erforderlich. Nach Beendigung der Natriumnitrit-Zugabe wird das Reaktionsgemisch bis zur vollständigen Nitrosierung etwa 1/2 bis 1 1/2 Stun den ständig gerührt, wobei die Zeitdauer bis zum vollständigen Ablauf der Nitrosierung vom pH-Wert, der Temperatur und dem Überschuß an Reaktionsmittel abhängig ist. Das Reaktionsprodukt, die Nitrosoverbindung, fällt als Niederschlag der Lösung aus.

Anschließend wird überschüssiges Nitrit durch Zugabe von Sulfaminsäure zerstört und der pH-Wert durch Zusatz eines alkalisch reagierenden Mittels, wie z.B. Natriumhydroxid, Natriumcarbonat, Kaliumhydroxid, Kaliumcarbonat, Lithiumhydroxid usw. auf einen Wert im alkalischen Bereich erhöht. Nachdem der pH-Wert des Reaktionsgemischs auf 8,0 bis 9,5, vorzugsweise auf 8,5 bis 9,5, eingestellt ist, löst sich die Nitrosoverbindung wieder auf.

Das erfindungsgemäße Verfahren wird fortgesetzt, indem die Nitrosoverbindung der aromatischen Sulfonsäure in das Sulfamat-Derivat umgewandelt wird (b). Die Lösung der Nitrosoverbindung kann zuvor noch gegebenenfalls durch Filtrieren oder Zentrifugieren gereinigt werden. Zur Herstellung des Sulfamat-Derivats wird die Nitrosoverbindung mit einem Metallsulfit, wie z.B. Natrium-oder Kaliumsulfit, vorzugsweise Natriumsulfit, umgesetzt. Das Metallsulfit kann als wäßrige Lösung zugesetzt werden, vorzugsweise wird es jedoch in fester Form eingebracht. Das Sulfit wird unter Rühren und gegebenenfalls ziemlich rasch zugegeben. Es bildet sich ein Nitroso-Sulfit-Addukt, das als Niederschlag aus der Lösung ausfällt, sich aber nach Erhitzen auf etwa 40-60° C wieder löst, wobei sich das Sulfamat-Derivat bildet.

Während der Bildung des Sulfamats soll der pH-Wert der Lösung durch Zugabe von Säure, wie z.B. Salzsäure oder Schwefelsäure, bei 9 bis 9,5 gehalten werden. Die Reaktionstemperatur wird anfangs vorzugsweise bei 45-50° C eingestellt; eine Überhitzung könnte zur Sulfonierung des Rings führen. Bei fortschreitender Reaktion beginnt der pH-Wert des Reaktionsgemischs zu steigen und muß deshalb durch Säurezugabe unter 9,5 gehalten werden. Zur Vervollständigung der Reaktion wird die Reaktionstemperatur schließlich auf 50-60° C, vorzugsweise auf 50-55° C, erhöht.

Nachdem sich der pH-Wert stabilisiert hat und dadurch das Ende der Umwandlung in das Sulfamat anzeigt, wird das Reaktionsgemisch auf 10-20° C abgekühlt und der pH-Wert auf 5,0 bis 6,0, vorzugsweise auf 5,5 bis 6,0, eingestellt. Anschließend wird die Sulfamatlösung mit einem Überschuß an Natriumnitrit versetzt und gut gemischt (d). Bei dem pH-Wert von 5,0 bis 6,0 erfolgt noch keine Reaktion. Wird die Lösung allerdings anschließend auf einen pH-Wert von etwa 3,0 angesäuert und die Temperatur bei höchstens 10° C gehalten, so bildet sich ein Niederschlag, der aus nicht umgesetzter Nitrosoverbindung, Aminonebenprodukten und anderen Verunreinigungen besteht. Durch Filtrieren oder Zentrifugieren können diese nicht gewünschten Verbindungen aus der Sulfamat-Nitrit-Lösung entfernt werden.

Die gereinigte Lösung wird dann durch Zugabe von Salzsäure bis auf einen pH-Wert von 1-1,5 noch weiter angesäuert und die Temperatur bei höchstens 10° C gehalten. Nach beendeter Säurezugabe wird die Reaktionstemperatur auf 15-20° C erhöht, wonach eine exotherme Reaktion einsetzt.

Nach Beendigung der exothermen Reaktion wird das Reaktionsgemisch bei einer Temperatur von 35-50° C, vorzugsweise 40° C, und einem pH-Wert unter 2,0, vorzugsweise 0,5 bis 1,0, gehalten. Falls erforderlich, wird noch einmal Nitrit zugegeben, so daß wegen des Überschusses die vollständige Diazotierung gewährleistet ist. Das Reaktionsgemisch wird dann auf etwa 10° C abgekühlt, mit einem inerten anorganischen Salz (z.B. Natriumchlorid) versetzt und die Lösung etwa 30 bis 60 min stehengelassen, wobei der pH-Wert bei etwa 1 gehalten wird. Die als Feststoff anfallende Aryldiazidsulfonsäure wird schließlich abgetrennt, gewaschen und getrocknet.

Das Mischen von Sulfamat und Diazotierungsreagenz entsprechend dem oben beschriebenen Reaktionsschema (c) vor dem Ansäuern der Reaktionsmischung kennzeichnet das erfindungsgemäße Verfahren im Besonderen, denn es wird dadurch sichergestellt, daß das Sulfamat, bevor es in Gegenwart von Säure in eine unlösliche, sehr schwer zu diazotierende Aminoverbindung hydrolisiert werden kann, in hoher Ausbeute in die gewünschte Aryldiazidsulfonsäure überführt wird. Produktausbeute und Produktreinheit sind deshalb unter Anwendung des erfindungsgemäßen Verfahrens überraschend hoch.

Ein weiterer Vorteil der Erfindung ist darin zu sehen, daß insbesondere in der anschließenden Reaktion (d), in der die Reaktionsmischung angesäuert wird, auch die durch die Reaktionsmaßnahme (c) nicht zu verhinderenden Nebenprodukte und Verunreinigungen in einfacher Weise durch Filtration oder Zentrifugation aus der Reaktionsmischung abgetrennt werden können, da diese auch hier als Feststoffe anfallen.

Die hohe Reinheit der entstehenden Aryldiazidsulfonsäure kann auch dadurch unterstützt werden, daß Ausgangsverbindungen gewählt werden, in denen mindestens ein Teil der bevorzugten Nitrosierungspositionen von Hydroxyalkylsulfonsäuren durch Substituenten, wie z.B. Sulfonsäuregruppen, niederen Alkylgruppen sowie Brom-, Chlor-oder Nitroatomen etc. blockiert werden.

Alternativ können nach dem erfindungsgemäßen Verfahren auch isomere Diazidgemische hergestellt werden, die als solche verwendet oder nach bekannten Verfahren in im wesentlichen reine Verbindungen aufgetrennt werden können.

Das erfindungemäße Verfahren kann diskontinuierlich, kontinuierlich oder halbkontinuierlich durchgeführt werden.

Die durch das erfindungsgemäße Verfahren hergestellten lichtempfindlichen Aryldiazidsulfonsäuren werden bevorzugt als lichtempfindliche Verbindungen in Photoresistgemische eingesetzt.

Das erfindungsgemäße Verfahren wird durch das folgende Reaktionsschema erläutert:

(a) 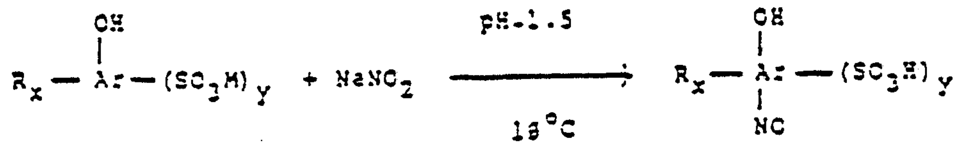


(b) 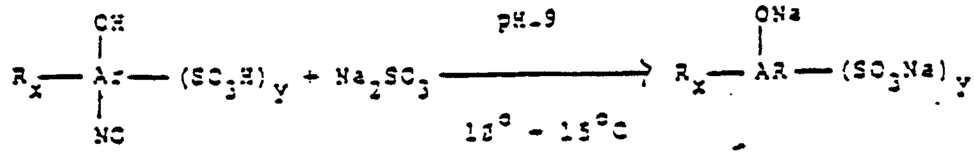


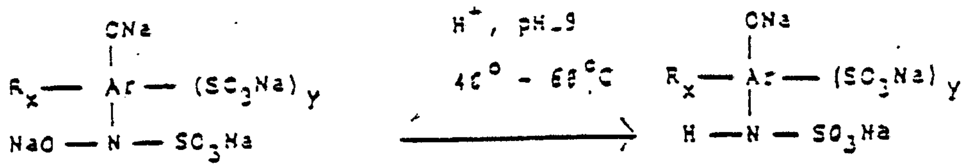


(c) 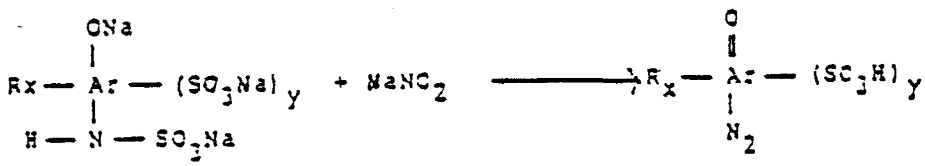

(d) pH → ~5.5 → ~3.8 → ~1.8

Temperatur → ~18°C → ~28°C → ~48°C

worin R, Ar, M, x und y die in den allgemeinen Formeln I und II angegebenen Bedeutungen haben.

Die Erfindung wird durch das folgende Beispiel näher erläutert, ohne darauf beschränkt zu werden:

Beispiel

Nevile-Winthersche Säure (56,0 g ≙ 0,250 mol, wenn 100%ig) wird in wäßriger Salzsäurelösung bei 10° C und einem pH-Wert von 1,5 bis 2,0 durch Zugabe eines Überschusses an 20 gew.%iger Natriumnitritlösung nitrosiert. Nach einer halben Stunde zerstört man überschüssiges Nitrit durch Zugabe von Sulfaminsäure und stellt den pH-Wert bei 10-15° C durch Hinzufügen von Natriumcarbonat auf etwa 9,0 ein, worauf sich die gebildete Nitrosoverbindung löst.

Über einen Zeitraum von 15 min wird bei pH 9,0 festes Natriumsulfit zugesetzt, wobei sich das Nitroso-Sulfit-Addukt bildet und als Niederschlag aus der Lösung ausfällt. Nach Erhitzen des Reaktionsgemischs auf 40-45° C bei pH 9,0 bis 9,5 fängt das Addukt an, sich zu lösen, und der pH-Wert beginnt von selbst zu steigen.

Die Temperatur wird so lange bei 42-48° C gehalten, bis der pH-Wert vorübergehend nicht mehr weiter ansteigt (etwa 1/2 bis 1 h). Während dieser Zeit bildet sich das Sulfamat unter weiterem Ansteigen des pH-Wertes in den alkalischen Bereich, so daß Salzsäure zugegeben werden muß, um den pH-Wert unter 9,5 zu drücken.

Das Reaktionsgemisch wird dann - falls erforderlich, unter Zugabe von weiterer Salzsäure, um den pH-Wert unter 9,5 zu halten - auf 50-55° C erhitzt und so lange in diesem Temperaturbereich gehalten, bis der pH-Wert nicht mehr von selbst ansteigt (etwa 10 min).

Nachdem die dunkelrote Sulfamatlösung auf 10-15° C abgekühlt und der pH-Wert durch Zugabe von Salzsäure auf 5,5 bis 6,0 eingestellt wurde, wird während eines Zeitraums von einer halben Stunde bei 10-15° C eine 20 gew.-%ige Natriumnitritlösung (etwa 3 mol $NaNO_2$ pro mol Nevile-Winthersche Säure)

zugegeben, wobei evtl. weitere Salzsäure zugegeben werden muß, um den pH-Wert bei 5,5 bis 6,0 zu halten; es können dabei Feststoffe aus der Lösung ausfallen. Anschließend wird bei 10° C der pH-Wert durch weitere Salzsäure auf etwa 3,0 herabgesetzt; die Farbe des Reaktionsgemischs wird heller, und es scheidet sich ein Niederschlag ab. Das Gemisch wird bei 10° C und dem pH-Wert 3,0 durch Filtration gereinigt, wobei das abgetrennte Nebenprodukt eine gelbgrüne Farbe aufweist.

Das gereinigte Filtrat wird gewogen und in ein temperierbares Becherglas mit pH-Meßelektrode zurückgegeben. Der pH-Wert wird auf 1,0 bis 1,5 herabgesetzt und die Temperatur auf 15-20° C erhöht. Wenn diese Bedingungen erreicht sind, steigt die Temperatur von selbst an, und der pH-Wert sinkt. Man läßt ohne Temperatursteuerung weiterreagieren. Bei 20 bis 25° C scheidet sich die Aryldiazidsulfonsäure in Form einer dicken Masse gelber Nadeln ab. Nach Beendigung der exothermen Reaktion wird das Reaktionsgemisch auf 40° C erhitzt und etwa 15 Minuten auf dieser Temperatur gehalten. Man gibt, falls erforderlich, weitere 20 gew.-%ige Natriumnitritlösung zu, hält den pH-Wert bei 0,5 bis 1,0 und prüft dabei, ob sich zusätzlich noch Nitrosogruppen bilden. Das Reaktionsgemisch wird dann auf 20-25° C abgekühlt und - bezogen auf sein Gewicht - mit 5 Gew.-% Natriumchlorid versetzt. Anschließend wird das Gemisch auf 5-7° C abgekühlt und - bezogen auf sein Gewicht - mit 5 Gew.-% Natriumchlorid versetzt. Anschließend wird das Gemisch auf 5-7° C abgekühlt und eine Stunde weitergerührt, wobei man den pH-Wert bei 0,5 bis 1,0 und die Temperatur auf 10° C hält.

Das Produkt wird dann mit Hilfe eines Büchner-Trichters abgetrennt und zweimal mit je einer kalten, angesäuerten 5-20 %-igen Natriumchlorid-Lösung gewaschen. Die Reaktion ergibt 57 g reine 1,2-Naphthochinon-2-diazid-4-sulfonsäure.

## Ansprüche

1. Verfahren zur Herstellung von lichtempfindlichen Aryldiazidsulfonsäuren ausgehend von mit mindestens einer Hydroxylgruppe substituierten Arylsulfonsäure, dadurch gekennzeichnet, daß während der Reaktionsabfolge, die aus

a) Nitrosierung der mindestens eine Hydroxylgruppe enthaltende Arylsulfonsäure,

b) Reduktion der entstehenden Nitrosoverbindung im alkalischen pH-Bereich und Umwandlung in ein Sulfamat-Derivat,

c) Mischen des Sulfamat-Derivates mit einem Diazotierungsreagenz und

d) Ansäuern dieser Mischung zur Bildung der Aryldiazidsulfonsäure

besteht, das nach jedem Reaktionsschritt entstehende und zur Weiterreaktion geeignete Reaktionsprodukt in Lösung verbleiben kann.

2. Verfahren nach Anspruch 1, dadurch gekennzeichnet, daß Arylsulfonsäuren der allgemeinen Formel I

$R_x$—A—$(SO_3M)_y$ ,     (I)

worin

A eine Arylgruppe mit einem Oxo-Anion (0=) und einem Diazonium-Kation ($N_2$=), z.B. des Chinon diazids oder des Naphthochinondiazids,

R einen einwertigen Substituenten, der ein Halogenatom, eine Nitrogruppe oder eine niedere Alkylgruppe mit 1 bis 6 C-Atomen sein kann,

M ein Wasserstoffatom, ein ein-, zwei-oder dreiwertiges Metall oder ein organisches Ammoniumion,

x eine ganze Zahl von 0 bis 3 und

y eine ganze Zahl von 1 bis 3

bedeuten.

3. Verfahren nach den beiden Ansprüchen 1 und 2, dadurch gekennzeichnet, daß die Reduktion der Nitrosoverbindung (b) bei einem pH-Wert von 8,0 bis 9,5 durchgeführt wird.

4. Verfahren nach einem oder mehreren der Ansprüche 1 bis 3, dadurch gekennzeichnet, daß das nichtlösliche Nitroso-Sulfit-Addukt bei einer Temperatur von 40-60° C in das lösliche Sulfamat-Derivat überführt wird (b).

5. Verfahren nach einem oder mehreren der Ansprüche 1 bis 4, dadurch gekennzeichnet, daß das Sulfamat-Derivat mit dem Diazotierungsreagenz unter Bedingungen, die nicht zur Reaktion führen, gemischt wird.

6. Verfahren nach Anspruch 5, dadurch gekennzeichnet, daß nach vollständiger Mischung der pH-Wert auf etwa 3 eingestellt wird.

7. Verfahren nach Anspruch 6, dadurch gekennzeichnet, daß die als Feststoffe entstehenden Nebenprodukte und Verunreinigungen durch Filtration oder Zentrifugation von der Reaktionslösung abgetrennt werden.